# EUROPEAN PATENT APPLICATION

(11) **EP 1 504 870 A2**
(43) Date of publication of application: **09.02.2005**
(21) Application number: 04018428.5
(22) Date of filing: 04.08.2004
(51) Int. Cl.: B29C 45/00, A61M 25/16

(54) **Catheter assemblies and injection molding processes and equipment for making the same**

(30) Priority: 08.08.2003 US 637836
(71) Applicant: Becton Dickinson and Company, Franklin Lakes, New Jersey 07417-1880 (US)
(72) Inventor: Fentress, James, Morrisville, North Carolina 27560 (US); O' Connor, Scott A., Durham, North Carolina 27713 (US); White, Scott A., Raleigh, North Carolina 27613 (US); Monahan, Lawrence A., Willow Spring, North Carolina 27592 (US); Martin, Frank E., Durham, North Carolina 27709-4042 (US); Tingey, Kevin G., Sandy, Utah 84092 (US); Gawreluk, Craig N., Park City, Utah 84098 (US)
(74) Representative: von Kreisler Selting Werner

(57) **Abstract**

Various single-part catheter assemblies, multi-part integrated catheter assemblies, and methods and apparatus for making the same are disclosed. The single-part catheter assemblies include a catheter hub integrally formed with a catheter tube for insertion into the bloodstream of a patient. The multi-part integrated catheter assemblies include a catheter hub integrally formed to a catheter tube for insertion into the bloodstream of a patient. The catheter assemblies of the invention may be injection molded in a single step or in multiple steps by injecting flows of molten plastic into a cavity of molds provided herein such that the flows converge into an even distribution about the circumference of the sleeve. The mold may have a core pin designed to fit into the cavity. Through the use of the even distribution of flows, the core pin may be seated within the cavity in an untensioned manner. The catheter assemblies of the invention may be produced of a single material or of multiple materials.

## Description

### BACKGROUND OF THE INVENTION

### 1. Field of the Invention

The present invention relates to medical systems and devices. More specifically, the present invention relates to catheter assemblies which include catheters and catheter adapters produced as a single part or produced as integrated assemblies of multiple parts. The invention further includes methods of injection molding and apparatus for producing such catheter assemblies from a single material and from multiple materials.

### 2. Description of Related Art

In an age of medicine in which injectable pharmaceuticals are ubiquitous in patient treatment regimens, indwelling catheters are a critical tool used in hospitals daily. Many medical treatments are dependent on devices and methods which allow the introduction of fluids into the body of a patient. Advances in catheter-related technologies have allowed a larger number of medical procedures to be performed intravenously instead of surgically. Indeed, procedures such as angioplasty and exploratory surgery may now be completed without making any incisions other than the puncture necessary to access a blood vessel and insert a catheter.

One type of commonly-used catheter is a peripheral intravenous catheter. These short, indwelling intravenous catheters are often used to provide an entry route for medications, fluid for hydration, and in some cases, for parenteral feeding. Such catheters are generally short in length, ranging from about one-half to about three inches in length. These catheters are generally made of flexible biocompatible materials. In some cases, these catheters additionally include a radiopaque compound such as barium sulfate to allow the location of the catheters to be tracked once inside the body.

Injection molding technologies have become very popular for use in producing plastic components, including many medical devices. The speed, efficiency, and consistency of these processes often results in time or cost savings to a manufacturer. Long, thin, tubular objects such as catheters have traditionally been very difficult to produce using such injection molding technologies. This difficulty arises for a number of reasons. First, the extremely high pressures involved in injection molding processes generally render proposed processes unusable. Typically, to provide a rapid fill, the molten plastic must be pressurized to several thousand pounds per square inch. As a result, when flows of the highly-pressurized plastic are allowed to enter the cavity of the injection mold, any imbalance in the flows, regardless of how slight, may deflect the core pin used to produce the tubular lumen of the component from its proper position. This often results in a damaged, and potentially unusable, product.

In answer to this problem, injection molding systems have been produced which have multiple gates through which the molten plastic is introduced. This results in multiple flows of molten plastic flowing into the mold. By providing multiple flows of molten plastic, imbalances in flow may generally be reduced. Despite this, however, flow imbalances may still occur. In many such cases these flow imbalances occur at least in part because the multiple flows may not enter the cavity in a simultaneous manner. In addition, imbalanced flows may occur when the flows of plastic are not evenly distributed about the core pin.

Another attempt to compensate for these factors has involved the production of injection molds and associated equipment which utilize a core pin which is placed in tension. Use of such systems and additional research have shown, however, that even in systems where the core pin is placed in high tension, imbalanced material flows may deflect the core pin, thus causing the part produced in the mold to have a number of undesirable characteristics. In many cases, these qualities include poor molecular orientation, excessive production of flash, internal stresses, and the like. In many cases, these flaws may be significant enough to impair the performance of the part or render it inoperative.

As a result of these difficulties, many thin tubular parts are produced using manufacturing processes such as extrusion which do not involve high-pressure injection-molding. Often, these alternate manufacturing procedures reduce ability of the producer to vary the shape, size, and overall geometry of the parts to be produced. As a result, thin tubular parts are first produced, and then in subsequent steps attached to other parts through separate processing steps. As discussed above, such post-production processing and the use of supplementary parts may be disadvantageous because of increased material and labor costs which may be associated with them.

Catheter assemblies comprising a catheter linked to a catheter adapter have typically been produced in a multi-step process such as those discussed above. Generally, the adapter portion is produced separately from the catheter portion and later attached. Following production of the components, the catheter is joined to the catheter adapter by threading the catheter into the adapter and attaching them to each other using a swage. In addition to this attachment step, the catheter portion may require a separate tipping process to provide a catheter with suitable tip geometry.

### SUMMARY OF THE INVENTION

The apparatus of the present invention has been developed in response to the present state of the art, and in particular, in response to the problems and needs in the art that have not yet been fully solved by currently available catheter assemblies and catheter assembly manufacturing methods and equipment. Thus, it is an overall objective of the present invention to provide injection-molded catheter assemblies including a catheter adapter and a catheter tube, as well as methods of manufacture and associated equipment by which such injection-molded catheter assemblies may be produced.

In accordance with the invention as embodied and broadly described herein, catheter assemblies are provided which include a catheter and an adapter either produced as a single component or separately in such a fashion that the fmal product is an integrally-joined assembly. The invention further provides methods and associated apparatus for producing these catheter assemblies by injection molding. The catheter assemblies of the invention include a catheter adapter or "catheter hub" and a catheter tube or "cannula." In some embodiments, the hub and cannula of the catheter assembly may be formed as a single component of a single material in a single step. In such a catheter assembly, the hub and cannula are continuous with each other, and are integrally formed. The invention includes methods and apparatus for producing such single-material one-piece catheter assemblies.

In other embodiments of the invention, the catheter assembly comprises a hub portion and a cannula portion which may be produced of different materials and in separate steps, but which are integrally attached to each other in the completed catheter assembly. This may generally involve producing one of the components first, and then subsequently overmolding the second component onto the first. In some embodiments, the hub is produced first and the catheter is subsequently overmolded. In others, the catheter portion is produced first and the hub is later molded about the catheter. The catheter assemblies of the invention may also be produced using injection molding methods in which the catheter and hub portions are produced substantially simultaneously.

In some embodiments, the hub portion and the cannula portion are attached to each other by bonds formed between the materials used to produce each portion during manufacturing. These bonds generally include non-covalent bonds such as electrostatic bonds, van der Waals interactions, or other chemical interactions or bonds. In addition, the hub portion and the cannula portion may be attached to each other by physical entanglement of the polymers used in the separate components.

In other embodiments, the hub portion and the cannula portion are attached to each other using a mechanical interlock interface created during manufacture of the components. In still other embodiments, the hub portion is attached to the cannula portion using a combination of the bonding of the materials, physical interaction of the polymers used in the components, and mechanical interlocking interfaces.

In the catheter assemblies of the invention, the hub portion of the catheter assembly is a generally tubular component used as an interface between the cannula portion and devices for introducing or withdrawing fluid from the body such as syringes. The hub portion includes a hub barrel, hub base, and a hub adapter. The hub barrel is the main tubular body of the catheter hub and includes a lumen for receiving a tip portion of a fluid withdrawal/introduction device such as a syringe or IV line, and for conveyance of fluid. The hub barrel is generally tapered internally and externally. The hub base protrudes from an end of the hub barrel in the form of a ridge. This ridge may further include flanges such as luer threads for locking the catheter assembly to an external device such as a syringe or an IV line in a secure, and often sealed, fashion. The flanges may also assist a user in grasping the hub barrel of the catheter assemblies of the invention.

The present invention also provides a method and related apparatus by which the single-material, single-part catheter assemblies of the invention may be manufactured through the use of injection molding processes. The invention provides a mold used for the injection molding of a single-material catheter assembly. This mold generally includes an A-side and a B-side which mate to form a cavity shaped to form a catheter assembly. The mold is configured to be coupled to a nozzle of a plastic injection system in order to receive a flow of molten plastic which is channeled to the cavity to form the catheter assembly. The A-side may have a cavity plate for receiving the flow or flows of molten plastic and for forming the catheter assembly. The B-side of the mold may have a floating plate for transmitting a flow or flows of molten plastic, as well as a base plate.

As briefly discussed above, the sides of the mold are configured to mate to produce a cavity into which plastic can be injected to form the catheter assembly. The cavity may be sealed in plastic-tight fashion such that gas can escape the cavity during inj ection, but plastic is unable to escape. A core pin generally protrudes into the cavity from the floating and base plates such that the cavity has a generally annular shape. The cavity may have a hub portion in which the hub is formed, and a catheter portion in which the catheter is formed. In molds for producing the single-material single-part catheter assemblies of the invention, the hub portions and catheter portions of the mold may be continuous with each other. The core pin may traverse the hub and catheter portions of the mold to define the lumen of the hub and the catheter. The cavity of the mold is further configured to provide a proper geometry to the tip of the completed catheter assembly.

The invention also provides methods and apparatus for forming the multi-material integrated catheter assemblies of the invention. Such methods include methods for producing an integrated two-piece (and hence potentially two-material) catheter assembly using two-shot or multi-shot injection molding techniques or using simple overmolding techniques. In each case, the mold is similar to that described above, with the exception that the cavity is configured to allow individual molding of either the catheter portion or the hub portion of the assembly first, and then to subsequently allow the molding of the remaining component or components about the first-molded part. This may be accomplished in a two-shot or multi-shot injection molding process by providing a modular cavity which segregates regions of the cavity until a first component has been molded, and then opens the remainder of the cavity to allow overmolding of the remaining part.

Similarly, overmolding techniques of providing a separate mold for the first component and a second mold configured to retain the molded first component and overmold the remaining one are taught. With overmolding, as above, either the catheter portion or the hub portion may be produced first, and then the remaining part subsequently overmolded about the first-produced part.

The molds of the invention may form the catheter assembly with a high degree of molecular alignment along its length by providing a comparatively even flow of molten plastic around the circumference of the annular cavity defining the catheter assembly. Such an even flow may be provided by providing a plurality of flows that converge and flow into the annular cavity substantially simultaneously.

In one example of this, floating plates of the molds of the invention may have a pair of substantially symmetrical flow paths through which molten plastic is able to travel from the nozzle to the hub portion of the cavity. Opposite sides of the hub portion of the cavity may have a gate region through which molten plastic emerges from the flow paths to enter the hub region of the cavity. The molten plastic may then travel through the hub in a substantially uniform manner. The molten plastic may thus fill the hub in a manner such that it is substantially evenly distributed about a circumference of the hub. From the hub, the plastic may enter the catheter portion of the cavity and move to the tip portion while maintaining an even distribution about the circumference of the cavity.

Thus, the two flows of molten plastic may reach the end of the tip portion simultaneously. Since molecules of molten plastic tend to align themselves with the direction in which the plastic flows, the result is a high degree of molecular alignment along the length of the catheter assembly, including the tip. The strength of the molded plastic part is generally greatest in the direction with which the molecules are aligned. Thus, the catheter assembly of the invention has a comparatively high resistance to axial tension and compression. In alternate methods, the molten plastic may be injected into the cavity such that it first fills the catheter portion of the cavity and then proceeds to fill the hub portion.

The use of even flows of molten plastic makes it unnecessary to employ extra steps to protect the core pin against bending. Some traditional injection molding processes utilize an external mechanism, such as a hydraulically operated clamp, to tension a core pin or other protrusion to form a bore in the injection molded part. Such mechanisms add to the complexity of the molding apparatus and increase the cycle time of the injection molding process, thereby increasing the cost of the injection molded parts. The present invention avoids this requirement, thus avoiding cost and reducing production time requirements.

After the plastic has been injected into the cavity, forming the catheter assembly, the mold may be disassembled to allow removal of the completed catheter assembly product. This often includes moving the B-side of the mold away from the A-side to expose the molded part. The completed catheter assembly may then be ejected from the core pin and cavity manually, by ejector pins, stripper plates, robotic removal, or other equipment and techniques known to one of ordinary skill in the art.

The cavity of the mold may be shaped such that the catheter assembly produced therein may have accurate tip geometry that promotes easier and more comfortable insertion of the catheter into a blood vessel. The catheter assembly may be rapidly and inexpensively manufactured by the injection molding process described above, without the need for separate attachment or tipping operations. Consequently, the catheter assembly and method of the present invention may contribute to the comfort, reliability, and cost effectiveness of medical care.

These and other objects, features, and advantages of the present invention will become more fully apparent from the following description and appended claims, or may be learned by the practice of the invention as set forth hereinafter.

### BRIEF DESCRIPTION OF THE DRAWINGS

In order that the manner in which the above-recited and other advantages and obj ects of the invention are obtained will be readily understood, a more particular description of the invention briefly described above will be rendered by reference to specific embodiments thereof which are illustrated in the appended drawings. Understanding that these drawings depict only typical embodiments of the invention and are not therefore to be considered to be limiting of its scope, the invention will be described and explained with additional specificity and detail through the use of the accompanying drawings in which:
Figure 1 is a perspective view of an embodiment of a single material catheter assembly of the invention;
Figure 2A is a perspective view of an embodiment of an integrated two-piece catheter assembly of the invention;
Figure 2B is a cross-sectional view of the integrated two-piece catheter assembly of Figure 2A;
Figure 3 is an exploded perspective view of the embodiment of the integrated two-piece catheter assembly of Figures 2A and 2B;
Figure 4 is an exploded perspective view of an alternate embodiment of the integrated two-piece catheter assembly of the invention;
Figure 5A is an exploded perspective view of another alternate embodiment of the integrated two-piece catheter assembly of the invention;
Figure 5B is an end view of the catheter hub of Figure 5A taken at line 5B-5B of Figure 5A;
Figure 6 is an exploded perspective view of another embodiment of the integrated two-piece catheter assembly of the invention;
Figure 7 is an exploded perspective view of yet another embodiment of the integrated two-piece catheter assembly of the invention;
Figure 8A is a perspective view of an embodiment of an integrated three-piece catheter assembly of the invention;
Figure 8B is a cross-sectional view of the integrated three-piece catheter assembly of Figure 8A;
Figure 9A is a perspective view of a mold for manufacturing single material catheter assemblies according to the invention;
Figure 9B is a partial plan view of the mold of Figure 8A taken at the line 8B-8B of Figure 8A;
Figure 10 is a cross-sectional view of the mold of Figure 8 showing a single material catheter assembly formed in the mold;
Figure 11 is a perspective view of a mold for producing the hub portion of an integrated two-piece catheter assembly according to the invention shown housing a completed catheter hub;
Figure 12 is an exploded perspective view of the mold of Figure 10 showing a catheter hub ejected from the mold;
Figure 13 is an exploded perspective view of a mold for overmolding a catheter portion onto the hub portion of the catheter assembly produced using the mold of Figures 11 and 12;
Figure 14 is a perspective view of the mold and catheter hub of Figure 13 showing a catheter tube portion overmolded onto the catheter hub;
Figure 15 is an exploded perspective view of the mold and the completed two-piece integrated catheter assembly of the invention formed using the mold of Figures 13 and 14; and
Figure 16 is a top cutaway view of an alternate mold of the invention for use in multi-shot injection molding methods of producing single- or multi-component integrated catheter assemblies according to the invention.

### DETAILED DESCRIPTION OF THE PREFERRED EMBODIMENTS

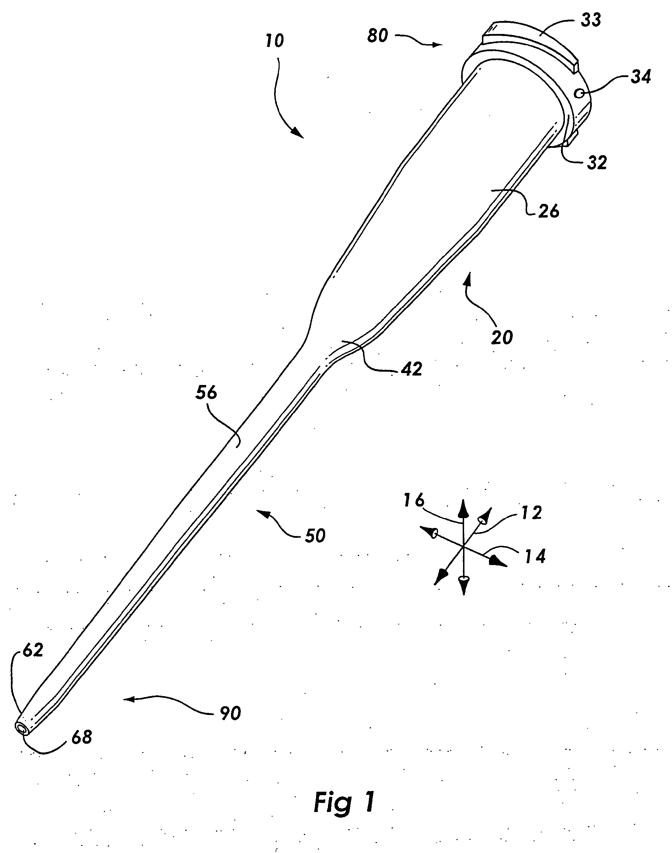

The presently preferred embodiments of the present invention will be best understood by reference to the drawings, wherein like parts are designated by like numerals throughout. It will be readily understood that the components of the present invention, as generally described and illustrated in the figures herein, could be arranged and designed in a wide variety of different configurations. Thus, the following more detailed description of the embodiments of the apparatus, system, and method of the present invention, as represented in Figures 1 through 16, is not intended to limit the scope of the invention, as claimed, but is merely representative of presently preferred embodiments of the invention.

The present invention includes advances in catheter design, material selection, and mold design which combine to enable production of a catheter assembly, including a catheter hub and a catheter tube, using injection-molding technologies. The invention further provides catheters which may be constructed using a single material, or using multiple materials. Among others, the invention provides single-material, bi-material, and tri-material integrally formed catheter assemblies and methods and molds for their **DETAILED**

### DESCRIPTION OF THE PREFERRED EMBODIMENTS

The presently preferred embodiments of the present invention will be best understood by reference to the drawings, wherein like parts are designated by like numerals throughout. It will be readily understood that the components of the present invention, as generally described and illustrated in the figures herein, could be arranged and designed in a wide variety of different configurations. Thus, the following more detailed description of the embodiments of the apparatus, system, and method of the present invention, as represented in Figures 1 through 16, is not intended to limit the scope of the invention, as claimed, but is merely representative of presently preferred embodiments of the invention.

The present invention includes advances in catheter design, material selection, and mold design which combine to enable production of a catheter assembly, including a catheter hub and a catheter tube, using injection-molding technologies. The invention further provides catheters which may be constructed using a single material, or using multiple materials. Among others, the invention provides single-material, bi-material, and tri-material integrally formed catheter assemblies and methods and molds for their the hub portion 20 of the assembly 10 of Figure 1, while the distal end 90 generally includes the catheter portion 50.

The hub 20 of the catheter assembly 10 of Figure 1 is configured to be attached to a variety of devices, including, but not limited to, syringes, IV lines, and other comparable devices. As such, the hub 20 may include flanges such as luer threads 33 mounted on a base 32 of the hub 20 which are configured to engage a luer lock system (not shown) to provide a fluid-tight connection between the assembly 10 and another device. The luer threads 33 may additionally assist a user in grasping the catheter assembly 10.

The hub portion 20 may further include a gate region 34 which corresponds to the region through which molten plastic was introduced into the mold used to form the catheter assembly 10. The position of this gate region 34 may be widely varied within the scope of the invention. In some embodiments of the catheter assembly 10, the hub 20 may include at least one gate region 34. In other embodiments of the catheter assembly 10, the hub 20 includes two or more such gate regions 34, indicating multiple flows of molten plastic into the mold used to form the catheter assembly 10. The gate regions 34 are generally positioned on the hub 20 of the assembly 10, and may be specifically located on the hub barrel 26, hub base 32, or luer threads 33. These gated regions 34 may appear as small bumps, small depressions, or even as surface irregularities on the surface of the hub 20 which are byproducts of the process of stripping the catheter assembly 10 from the mold used to produce it. The position of the gated regions 34 may be specified to optimize the function and appearance of the catheter assembly 10. The function and origin of these gate regions 34 and the mold used to produce the catheter assembly 10 will be described in greater detail below.

The hub 20 of the catheter assembly 10 further includes a hub barrel 26 which extends in a longitudinal direction 12 from the hub base 32. At an end of the hub barrel 26 opposite the hub base 32, the hub region 20 joins a transition region 42 which has a shape that links the generally wider catheter hub region 20 to the catheter region 50 of the catheter assembly 10. The hub portion 20 further includes a hub lumen (not shown) that runs the length of the hub portion 20, and which is continuous with an interior lumen 68 of the catheter region 20.

The catheter region 50 of the catheter assembly 10 joins the hub portion 20 at the transition region 42. The catheter region 50 extends outwardly in a longitudinal direction 12 from the transition region 42 in the form of a narrow tubular catheter cannula 56. The catheter 56 is integrally formed with the hub region 20 of the catheter assembly 10. The catheter portion 50 includes a catheter lumen 68 which is in fluid communication with the lumen (not shown) of the hub portion 20. The length of the catheter 56 of the assembly 10 may be varied widely within the scope of the invention, but is generally from about one-half to about three inches in length. The catheter 50 terminates in a tip 62. The geometry of the tip 62 may be varied within the scope of the invention.

The tip 62 of the cannula 56 of the catheter 50 may be configured to have any of a variety of geometries to facilitate its insertion into the bloodstream of a patient. The catheter assembly 10 is generally inserted into a bloodstream by placing the catheter assembly 10 over a needle, catheter introducer or other similar device, pushing the needle through the skin of the patient, and withdrawing the needle, thus leaving the catheter assembly 10 in place for use in withdrawing fluid or introducing fluid into the bloodstream of the patient.

The catheter assembly 10 may be designed and produced such that the hub portion 20 and the catheter portion 50 both include at least a slight draft angle, so that the hub portion 20 and the catheter portion 50 are each slightly wider at their proximal ends than at their distal ends. This draft angle may be varied to aid in steps of the production of the catheter assembly 10, including removal of the finished product from a mold.

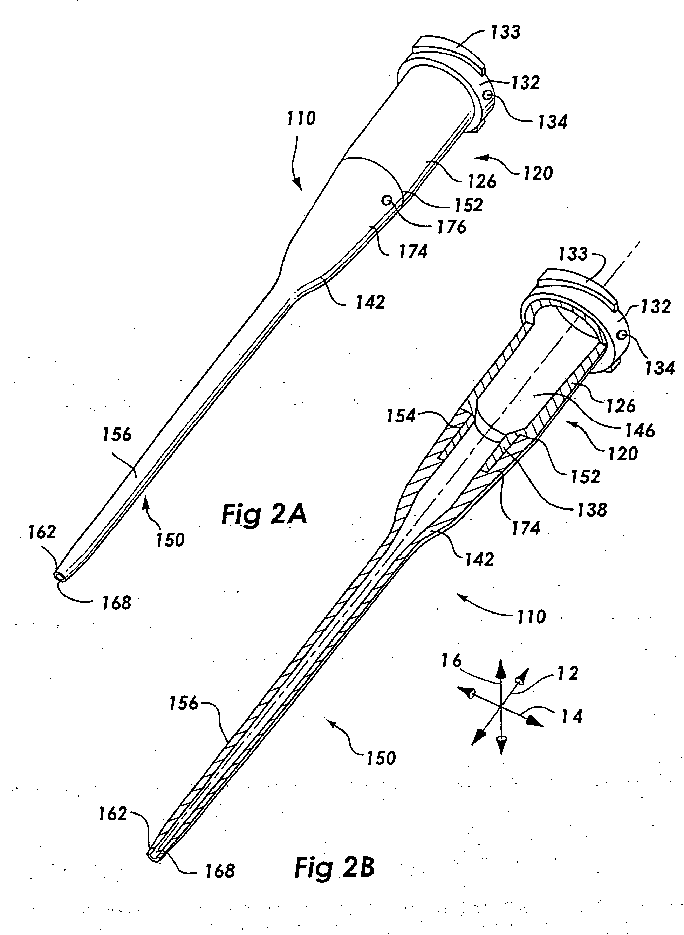

Referring now to Figure 2A, another embodiment of the catheter assembly 110 of the invention is shown. This embodiment of the catheter assembly 110 of the invention includes at least two integrally-formed components, and thus may be constructed of more than one material. Specifically, the catheter assembly 110 includes a hub portion 120 and a catheter portion 150 which merge at a joint 152. In this embodiment of the catheter assembly 110, the hub portion 120 includes a hub base 132 including luer threads 133, a hub barrel 126 attached to the hub base, and a hub adapter (not shown) that interfaces with the catheter portion 150 of the assembly 110. The catheter portion 150 includes a tip 162, a catheter cannula 156, a transition region 142, and an attachment sleeve 174.

As above, the hub portion 120 may include a gate region 134. In other embodiments of the catheter assembly 110, the hub 120 includes two or more such gate regions 134. In this embodiment, the gate region 34 is shown positioned on the hub base 132 of the assembly 110. The catheter portion 150 of the catheter assembly 110 may also include a gate region 176 on its surface. In some embodiments, the gate region 176 may be positioned on the attachment sleeve 174 of the catheter region 150, as shown in Figure 2A. In other embodiments, the gate region 176 may be positioned on the transition region, or alternatively, on the catheter cannula 156.

The joint 152 of the catheter assembly 110 is shown in cross-sectional detail in Figure 2B. In the catheter assembly 110, the catheter portion 150 attaches to the hub portion 120 via the attachment sleeve 174 extending from the transition region 142 of the catheter portion 150. As seen in Figure 2B, the attachment sleeve 174 projects outwardly from the transition region 142 of the catheter portion 150. The attachment sleeve 174 has a geometry configured to surround and interface with the hub adapter 138 of the hub portion 120 of the assembly 110 to provide attachment at the joint 152 in a substantially sealed manner. This sealed attachment at the joint 152 may be provided in a variety of ways.

The joint 152 may be produced relying upon a variety of factors to produce a strong, sealed joint 152. First, the materials used to produce the hub and catheter portions 120, 150 may be selected such that the materials will adhere to each other during the manufacturing process. Examples of materials having suitable characteristics will be discussed in greater detail below. As discussed briefly above, the materials selected for the hub and catheter portions 120, 150 may be selected to give each individual portion a physical property that may be desirable. In some embodiments, it may be desirable to produce the hub portion 120 out of a material demonstrating rigidity to assist in attaching the assembly 110 to other equipment. In addition, in some embodiments, it may be desirable to produce the catheter portion 150 out of a substantially flexible material to allow insertion and use of the catheter 150 without damage to the catheter 150 or injury to the circulatory system of the patient.

As above, the catheter assembly 110 may be designed and produced such that the hub portion 120 and the catheter portion 150 both include at least a slight draft angle, so that the hub portion 120 and the catheter portion 150 are each slightly wider at their proximal ends than at their distal ends. This draft angle may be varied to aid in steps of the production of the catheter assembly 110, including removal of the finished product from a mold.

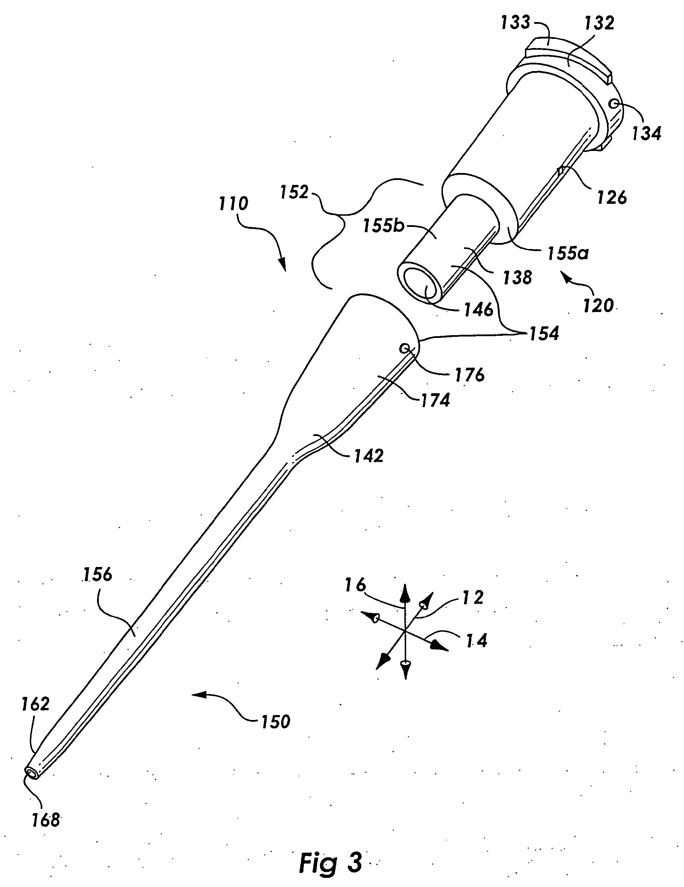

Referring now to Figure 3, an exploded view of the catheter assembly 110 of Figures 2A and 2B is shown. Specifically, Figure 3 shows the catheter assembly 110 separated along the interface 154 of the joint 152 of the hub portion 120 and the catheter portion 150 of the assembly 110 of Figures 2A, 2B. Figure 3 illustrates the conformation of the surfaces of the hub adapter 138 and the attachment sleeve 174 that form the interface 154 of the joint 152.

The strength of the joint 152 of the catheter assembly 110 of Figure 3 may be modulated by varying the size of the interface 154 of surfaces of the catheter portion 150 and the hub portion 120 of the catheter assembly 110. In embodiments using materials which adhere to each other during the manufacturing process, increasing the surface area of the interface 154 of the joint 152 increases the strength of the joint 152. Thus, in the catheter assembly 110, the length of the attachment sleeve 174 and the length of the corresponding hub adapter 138 may be varied to change the total surface area of the interface 154. This, in turn, varies the strength of the resulting joint 152.

The interface 154 of the joint 152 of the catheter assembly 110 may be configured to provide a tensile strength zone 155a and a shear strength zone 155b. Specifically, the tensile strength zone 155a may be defined as the surface area of the ring shown at the interface 154. This surface area may be varied by extending or reducing the radii of the interior and exterior of the hub portion 120. When the hub 120 and the catheter 150 are made of compounds which chemically bond, e.g. via electrostatic bonds, van der Waals interactions, or other chemical interactions or bonds; or when the hub 120 and catheter 150 are made of compounds such as polymers which my physically entangle, this may increase or reduce the tensile strength of the joint 152.

The shear strength zone 155b is the surface area of the sleeve projecting in a longitudinal direction 12 from the tensile strength zone 155a. The area of the shear strength zone 155b may similarly be varied by extending the longitudinal length of the shear strength zone 155b, or by expanding its radius. When the hub 120 and the catheter portion 150 are made of compounds which bond in any of the manners described above, this may increase or decrease the strength of the joint 152. These principles may be applied in many of the embodiments of the two-piece integrated catheter assemblies of the invention to provide adequate tensile and shear strength with various pairs of compounds used to form the individual components of the catheter assembly 110.

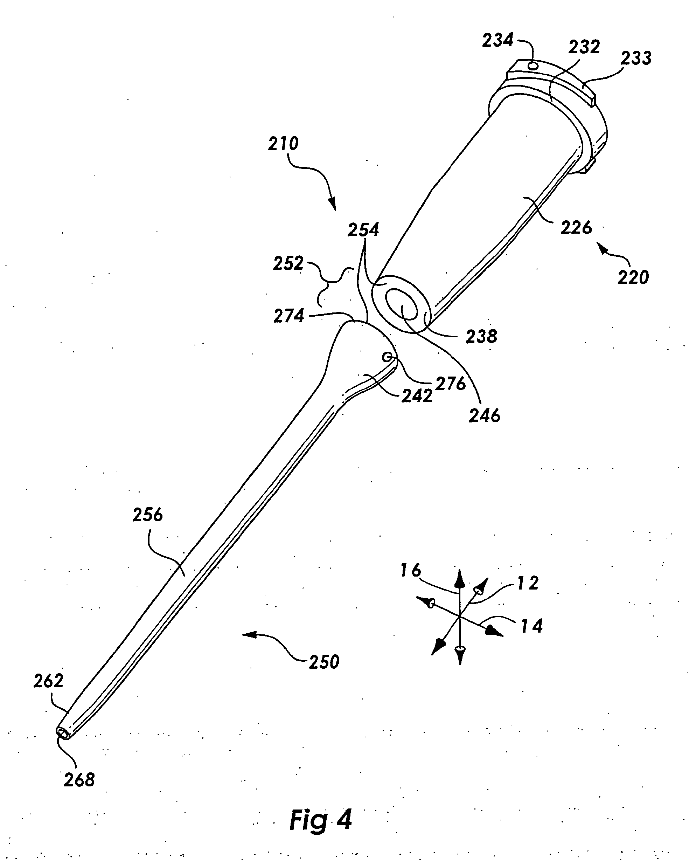

Figure 4 shows an exploded perspective view of additional embodiment of a catheter assembly 210 of the invention. Similar to the assembly 110 of Figures 2A-3, the embodiment of Figure 4 includes a hub portion 220 and a catheter portion 250. The hub portion 220 includes a hub base 232 which may include luer threads 233 to allow locking the assembly 210 to a compatible device in a fluid-sealed manner. The hub portion 220 further includes a hub barrel 226 extending from the hub base 232. In this embodiment, the hub barrel 226 terminates in a hub adapter face 238. The hub 220 further includes a gate region 234 positioned on the luer threads 233 of the hub base 234. In use, the hub adapter face 238 is attached to the catheter portion 250 of the assembly 210.

The catheter portion 250 of the assembly 210 has a catheter attachment face 274 configured to be joined to the hub adapter face 238. The catheter portion 250 further includes a gate region 276 positioned on the transition region 242. In this embodiment of the assembly 210, the attachment of the catheter attachment face 274 and the hub adapter face 238 forms a butt-joint 252. In this embodiment of the catheter assembly 210, the butt joint 252 includes an interface 254 having a surface area which may be smaller than that of the catheter assembly 110 of Figures 2A-3. In embodiments of the invention such as the assembly 210 shown in Figure 4, the strength of the joint 252 formed between the hub portion 220 and the catheter portion 250 is dependent on the strength of the bond formed between the materials used to produce the specific portions 220, 250. Indeed, the materials used to produce the specific portions 220, 250 may be selected for the strength of the bond they form since the surface area of the interface 254 may be much lower than that of the surface area of the interface of the assembly 110 of Figures 2A-3.

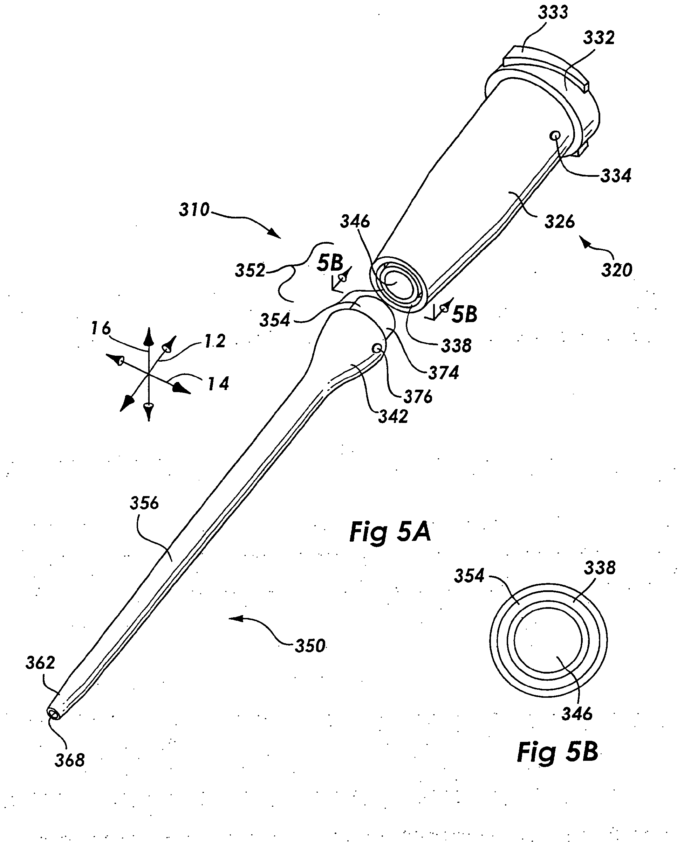

Referring now to Figure 5A, yet another embodiment of a catheter assembly 310 of the invention is shown in an exploded perspective view. The catheter assembly 310 includes a hub portion 320 and a catheter portion 350. The hub portion 320 includes a hub base 332 which may include luer threads 333 to allow locking the assembly 310 to a compatible device in a fluid-sealed manner. The hub portion 320 further includes a hub barrel 326 extending in a longitudinal direction 12 from the hub base 332. The hub barrel 326 includes a gate region 334. The hub barrel 326 terminates in a hub adapter face 338. In use, the hub adapter face 338 is attached to the catheter portion 350 of the assembly 310. The catheter portion 350 of the assembly 310 includes an attachment sleeve 374 extending from the transition region 342, and a catheter cannula 356 terminating in a tip 362. The catheter portion 350 also includes a gate region 376, positioned on the transition region 342. The catheter portion 350 also has a lumen 368 which is continuous with the lumen 346 of the hub portion 320 of the catheter assembly 310 when assembled.

The hub adapter face 338 of the catheter assembly 310 is configured as an internal interlock sleeve joint to allow use of a relatively short attachment sleeve 374. Despite their short length, the hub adapter face 338 and the attachment sleeve 374 form an interface 354 having a relatively large surface area because both the inward-facing and outward-facing surfaces of the attachment sleeve 374 interface with the hub adapter face 338. Various other structural conformations of the attachment sleeve 374 and the hub adapter 338 may be used within the scope of the invention to provide a large surface area for the interface 354 of the joint 352.

Referring now to Figure 5B, an end view of the hub 320 taken from line 5B-5B is shown. This end view shows the hub adapter face 338 of the catheter assembly 310. This hub adapter face 338 includes an interface 354. The hub lumen 346 is also shown. In Figure 5B, the interface 354 is an annular groove configured to receive the attachment sleeve 374 shown in Figure 5A.

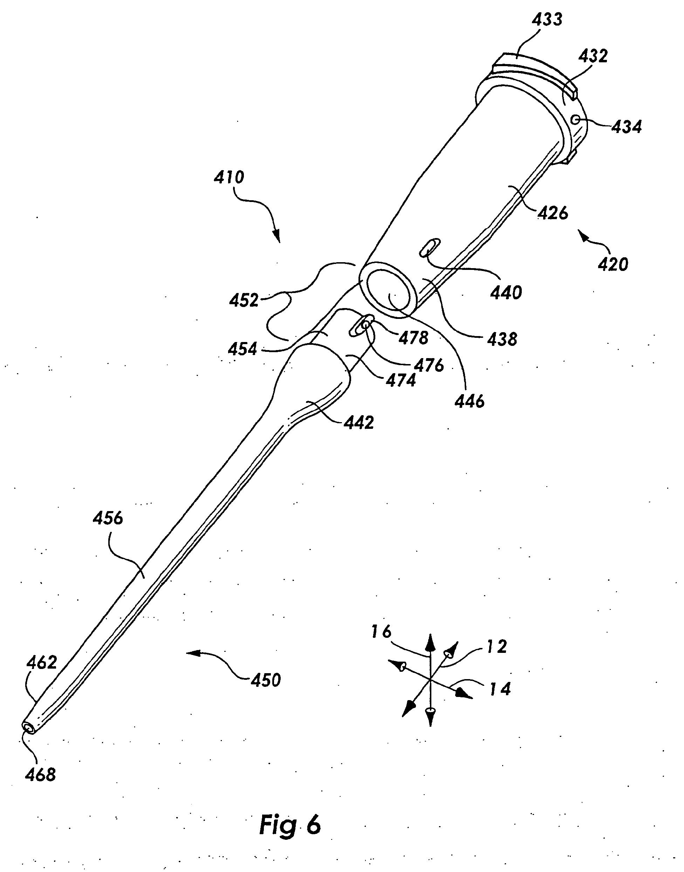

The strength of the joints 152, 252, 352 of embodiments of the invention shown in Figures 2 through 5B may be further modified by incorporating features into the interfaces 154, 254, 354 which provide a mechanical interlock. Such mechanically-interlocking features may be provided alone, or may be provided to enhance the strength of chemical bonds formed between the materials at the joint 152, 252, and 352. One such embodiment of a catheter assembly 410 that includes a mechanical interlock is shown in Figure 6.

Referring now to Figure 6, another catheter assembly 410 according to the invention is shown. The catheter assembly 410 again includes a hub portion 420 and a catheter portion 450. The hub portion 420 includes a hub base 432 which may include luer threads 433 to allow locking the assembly 410 to a compatible device in a fluid-sealed manner. The hub base 432 further includes a gate portion 434. The hub portion 420 further includes a hub barrel 426 extending from the hub base 432. The hub barrel 426 terminates in a hub adapter 438. The hub adapter 438 here includes a locking orifice 440 which integrates with the catheter portion 450.

The catheter portion 450 of the assembly 410 includes an attachment sleeve 474 extending from the transition region 442. In this embodiment, the attachment sleeve 474 includes a gate region 476. The catheter assembly 410 of Figure 6 shows one version of a joint 452 which utilizes a mechanical interlock. In this embodiment of the catheter assembly 410, the attachment sleeve 474 includes an attachment lock 478 in the form of a raised peg 478 configured to pass through the locking orifice 440 of the hub adapter 438. The gate region 476 may be positioned on the mechanical interlock 478 as shown in Figure 6, or may be located on other regions of the catheter portion 450 to ease manufacturing of the assembly 410. The positioning of the attachment lock 478 through the locking orifice 440 as the lock 478 is produced provides a mechanical interlock. Thus, the interface 454 between the hub portion 420 and the catheter portion 450 is adapted to provide a more strong attachment.

As in previous embodiments, the catheter portion 450 of the assembly 410 further includes a catheter cannula 456, also extending from the transition region 442, which terminates in a tip 462. The catheter portion 450 also has a lumen 468 which is continuous with the lumen 446 of the hub portion 420 of the catheter assembly 410 when assembled.

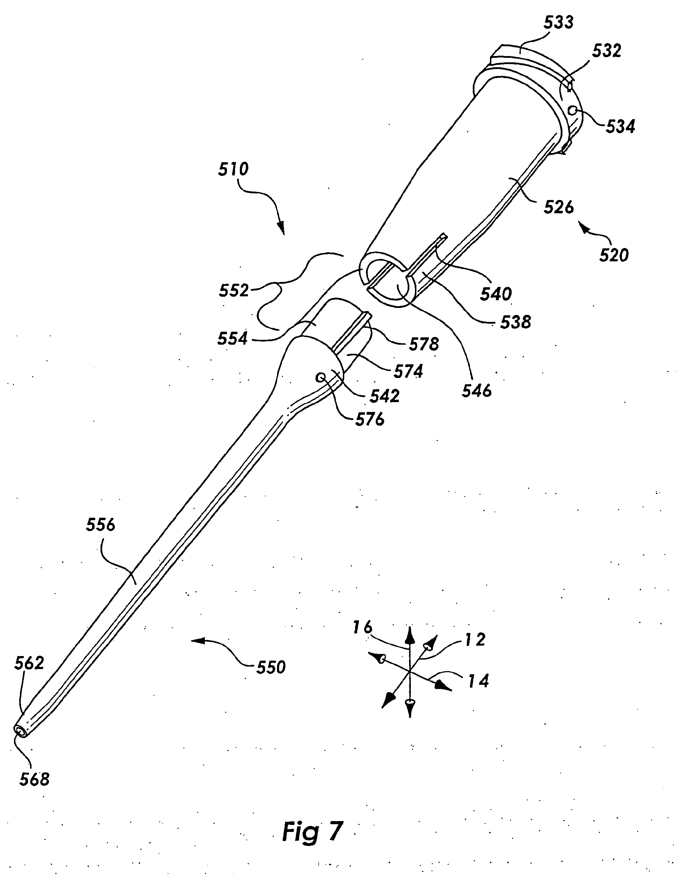

Referring next to Figure 7, yet another catheter assembly 510 of the invention is shown. The catheter assembly 510 includes a hub portion 520 and a catheter portion 550. The hub portion 520 includes a hub base 532 which may include luer threads 533 to allow locking the assembly 510 to a compatible device in a fluid-sealed manner. The hub base 532 here further includes two gate regions 534 (only one of which is visible). The hub portion 520 further includes a hub barrel 526 extending from the hub base 532. The hub barrel 526 terminates in a hub adapter 538. The hub adapter 538 here includes a locking slot 540 which integrates with the catheter portion 550.

The catheter portion 550 of the assembly 510 includes an attachment sleeve 574 extending from the transition region 542. The catheter portion 550 here includes two gate regions 576 (only one of which is visible) which resulted from the production methods used to produce in the assembly 510. The catheter assembly 510 of Figure 7 shows another version of a joint 552 which utilizes a mechanical interlock to provide strength to the joint 552. In this embodiment of the catheter assembly 510, the attachment sleeve 574 includes an attachment ridge 578 configured to fit in the locking slot 540 of the hub adapter 538. The fit of the attachment ridge 578 into the locking orifice 540 provides a mechanical interlock.

Referring next to Figure 8A, yet another catheter assembly 910 of the invention is shown in a perspective view. This catheter assembly 910 is a multi-component catheter assembly comprising a hub portion 920, a flexible pivot portion 980, and a catheter portion 950. The hub portion 920 includes a hub base 932 which may include luer threads 933 to allow locking the assembly 910 to a compatible device in a fluid-sealed manner. The hub base 932 here further includes two gate regions 934 (only one of which is visible). The hub portion 920 further includes a hub barrel 926 extending from the hub base 932. The catheter portion 950 of the assembly 910 includes a transition region 942. The catheter portion 950 here includes two gate regions 976 (only one of which is visible) which resulted from the production methods used to produce in the assembly 910.

In this embodiment of the catheter assemblies of the invention, the catheter assembly 910 further comprises a flexible pivot portion 980. The flexible pivot 980 is positioned between the hub 920 and the catheter 950 and interfaces with them at joints 952a, 952b. The flexible pivot 980 is configured to allow manipulation and movement of the hub portion 920 of the catheter assembly 910 while minimizing disturbance or displacement of the catheter portion 950 of the catheter assembly 910.

In the catheter assembly 910 of Figure 8A, the flexible pivot 980 includes ridges 982. These ridges 982 act as accordion-pleats to allow easy bending of the pivot 980. In addition, as discussed in greater detail below, the pivot portion 980 may be produced of a material which is flexible and resilient in order to allow bending and return to its original shape. The provision of the flexible pivot 980 to the catheter assemblies of the invention allows placement of the catheter portion 950 of the assembly 910 and subsequent manipulation of the hub portion 920 without disturbing the placement of the catheter portion 950 in a patient.

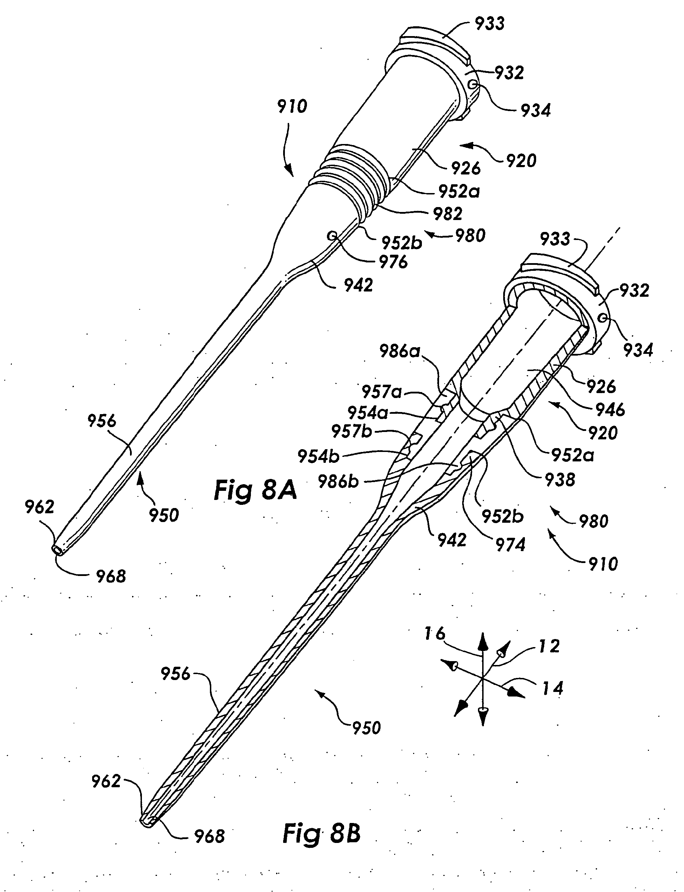

Figure 8B shows a partially-cut-away perspective view of the catheter assembly 910 of Figure 8A. As above, the assembly 910 includes a hub portion 910, a catheter portion 950, and a flexible pivot 980. In Figure 8B, the assembly is shown partially cut away to reveal the lumen 946 of the hub portion 920 and the lumen 968 of the catheter portion 950. Figure 8B also demonstrates one possible configuration of the joints 952a, 952b linking the hub 920 and the catheter 950 to the flexible pivot 980. As previously discussed, the joints 952a, 952b may simply rely on the bonding properties of the materials used to form each of the segments 920, 950, 980 of the catheter assembly 910.

The three-component configuration of the catheter assembly 910 adds further flexibility in the design of the catheter assemblies of the invention. Specifically, in the two-component assemblies 110, 210, 310, 410, and 510 discussed previously, the materials of the hub and catheter portions were generally selected for their ability to bind to each other. In the three-component configuration of the catheter assembly 910, the materials of the hub 920 and catheter 950 may not bond well to each other, but may instead bond well to the material used to form the flexible pivot 980 of the assembly 910.

The pivot 980 of the catheter assembly 910 of Figure 8B is joined to the hub 920 and catheter 950 portions of the assembly 190 at joints 952a, 952b. Each of these joints 952a, 952b relies on the bonding of the materials at interfaces 954a, 954b, and on a mechanical interlock. More specifically, each of the joints 952a, 952b, comprises a circumferential rib 957a, 957b. The joint 952a is formed by a hub adapter 938 interfacing at 954a with a first pivot attachment 986a. At joint 952a, the hub adapter 938 comprises a circumferential rib 957a which is received by the pivot attachment 986a. The joint 952b is formed by an attachment sleeve 974 interfacing at 954b with a second pivot attachment 986b. At joint 952b, the second pivot attachment 986b comprises a circumferential rib 957b which is received by the attachment sleeve 974.

As discussed previously, the size and geometry of the interfaces 954a, 954b may be varied to vary the strength of the bond. In addition, the specific type of mechanical interlock used in catheter assemblies such as 410, 510, and 910 may be varied by one of skill in the art. Those of skill in the art will recognize that numerous other catheter assemblies may be made within the scope of the present invention.

Various materials have been tested for suitability for use in producing the catheter assemblies of the invention. Several components have been tested for use in producing the single-part, single-material catheter assemblies such as 10, and the catheter portions of multi-part catheter assemblies such as 110, 210, 310, 410, 510, and 910. These complete catheter assemblies and catheter portions have been produced of polyurethane materials. In specific embodiments of the catheter assemblies of the invention, a low viscosity polyurethane is desirable. In some specific embodiments of the invention, a proprietary polyurethane Vialon™ is used.

Vialon™ is a polyurethane biomaterial used in catheter products. Vialon™ may be molded to produce a smooth surface which reduces catheter drag during insertion and prevents catheter-related thrombosis during use as an indwelling catheter device. Catheter products produced with Vialon™ soften after insertion into the circulatory system of a patient, allowing the catheter to better conform to the natural shape and form of the blood vessel into which it has been inserted. This helps to reduce injury to or irritation of the lining of the vessel.

In specific embodiments of the invention, the catheter portion 150 is produced using Vialon™ modified to reduce its viscosity. The process used to modify the viscosity of the Vialon™ may include reducing the molecular weight of the Vialon™ using multiple: pass extrusion.

Other materials are also suitable for use in producing the single-part single-material catheter assemblies (such as 10 of Figure 1), and the catheter portions of multi-part catheter assemblies such as 110, 210, 310, 410, 510, 910. Suitable materials include polyurethane elastomer, polyester, polyethylene, polypropylene, polybutylene, polytetrafluoroethylene, fluorinated ethylene-propylene copolymer, silicone, polyether block amides ("PEBAX"), and poly vinyl chloride.

Several materials have been tested for use in the hub and catheter portions of the catheter assemblies (such as 110, 210, 310, 410, 510, and 910) of the invention. In some embodiments, the hub portion has been produced of polycarbonate and polyurethane materials. In addition to these materials, however, PET, nylon 12 homopolymer, and nylon 12 copolymer have been tested. The resulting experimental data indicates that they would be suitable for use in producing the catheter hub 120 of the assemblies 110 of the invention. Specifically, such materials provide a relatively rigid base to allow for secure attachment of the assembly 110 to outside apparatus.

In addition to the above materials, other materials have been tested for use in the hub and catheter portions of the catheter assemblies (such as 110, 210, 310, 410, 510, and 910) of the invention. In some embodiments, the hub portion may be produced of materials such as nylon, polymethyl-methyacrylate, polyester, acrylo-nitrile butadiene styrene, polyurethane, polyethylene, polypropylene, polyether block amides ("PEBAX"), poly vinyl chloride, polycarbonate, acrylic, polystyrene, and polymethylpentene.

The catheter assemblies of the invention may alternatively be produced using reaction injection molding technologies, in which prepolymers are injected into the mold instead of using molten polymeric materials. After injection, the prepolymers polymerize and cure to form the completed catheter assemblies of the invention. Reaction injection molding processes often operate at temperatures lower than those required for traditional injection molding technologies. This may also reduce the energy expenditures required to produce the catheter assemblies of the invention. In addition, since prepolymers are generally less viscous than molten polymers, they may flow more easily into molds, reducing tooling costs. This may make reaction injection molding useful in catheter assembly designs using complex joints or tip geometries. Reaction injection molding cycle times are also generally short, resulting in cycle times of less than about one minute. Materials suitable in reaction injection molding methods generally include polyurethanes, nylons, and other fast-reacting prepolymers. Generally, amine-extended polymers form more quickly, while diols may require up to 60 seconds for sufficient polymerization.

Material bond strength studies were conducted using various combinations of several of the above-listed materials to produce catheter assemblies according to the catheter design of Figure 4, having a relatively small interface area. In these studies, the predicted loads for failure of a catheter constructed using the following adapter materials and either clear or filled Vialon™ were determined. These predicted failure loads are shown in Table 1 below:

| **TABLE 1** | **Predicted Load for Failure of Adapter/Tube Interface (1b) End-joint Design** | |
|---|---|---|
| **Adapter Material** | **Clear Vialon**^{**TM**} | **Filled Vialon**™ |
| PET | 10 | 6.8 |
| Polycarbonate (Lipid Resistant) | 13 | 4.0 |
| Polycarbonate | 35 | No data available |
| Isoplast | 38 | 25 |
| Nylon 12 Homopolymer | 33 | 30 |
| Nylon 12 Copolymer | No joint failure | 17 |

Although predicted failure loads for the first two materials appear less favorable, catheter designs providing a larger interface/contact area may likely be suitable. In addition, it is believed that Vialon™ having less of the radiopaque compound (here barium sulfate), and thus, adhesion will be improved.

Additional data were obtained from materials testing studies. The data obtained is shown in Table 2 below.

| **TABLE 2** | **Stress at Failure (psi)** | |
|---|---|---|
| **Adapter Material** | **Clear Vialon** | **Filled Vialon** |
| PET | 646 | 436 |
| Polycarbonate (Lipid Resistant) | 804 | 251 |
| Polycarbonate | 2260 | No data available |
| Isoplast | 2450 | 1581 |
| Nylon 12 Homopolymer | 2090 | 1930 |
| Nylon 12 Copolymer | No joint failure | 1080 |

In these materials testing studies, catheters were produced with either Vialon™ or Vialon™ including a radiopaque compound, referred to herein as "filled VialonTM" and a second adapter material. These catheters were then subjected to pull-testing to test adhesion quality. It is believed that the stess at failure exhibits sufficient adhesion to resist a 3-pound pullout force.

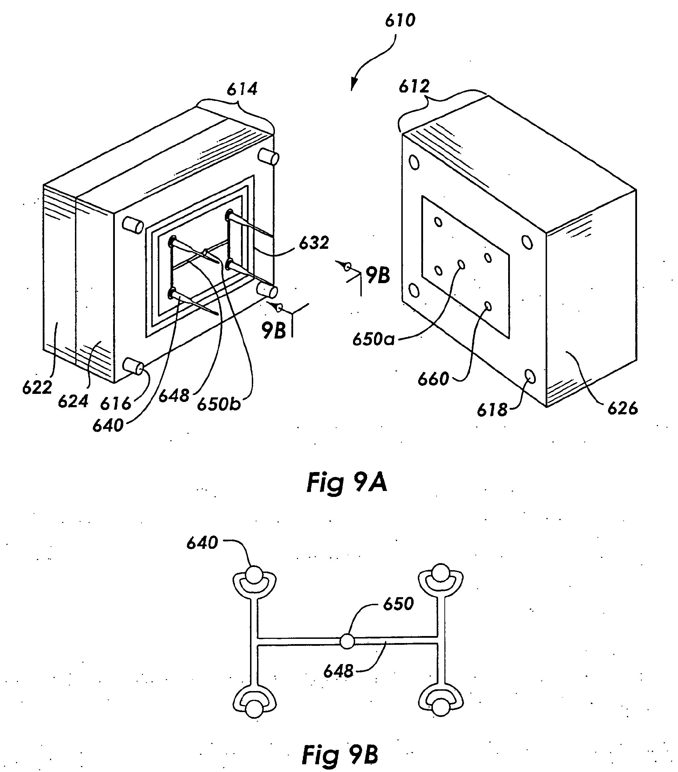

The following discussion returns to the catheter assembly 10 of Figure 1 to describe example of methods by which the catheter assemblies of the invention may be produced. In a first set of methods, the catheter assembly 10 may be produced using injection molding. Referring now to Figure 9A, a mold is shown for producing single-component, single-material catheter assemblies of the invention such as catheter assembly 10 of Figure 1. The catheter assembly 10 of Figure 1 may be manufactured in a "one step" fashion. In this application, the term "one step" in the context of manufacturing refers to a process of completely forming an item in a substantially final, usable condition, with a single manufacturing process. Manufacturing processes such as injection molding may, itself, have several discrete steps, however, if operations such as core pin tensioning, "tipping" (insertion of the tip into a specialized tip mold or other processing of the completed catheter assembly to provide a catheter with an acceptable tip geometry), part attachment, or other secondary molding steps are avoided, the process is still referred to herein as a "one step" process.

Referring now to Figure 9A, one embodiment of a mold 610 capable of molding the catheter assembly 10 of Figure 1 in a one step manner is shown. The mold 610 may be used or adapted for use with a wide variety of injection molding machines. Suitable injection molding machines used with the mold 610 are capable of providing rapid and accurate acceleration and deceleration of a selected molten plastic into the mold 610 to ensure that the mold 610 is properly filled. Further, suitable injection molding machines are generally capable of doing this rapidly and repeatedly while exhibiting consistent performance in each iteration of the manufacturing process. The injection molding machine has been omitted from Figure 9A for clarity.

The mold 610 may have an A-side 612 that may be coupled to an injection nozzle of the injection molding machine. In embodiments in which the nozzle is coupled to the A-side 612, the nozzle is attached so as to be continuous with an orifice 650a configured to receive molten plastic. The molten plastic may then be channeled to the cavities 660 of the A-side 612 via a region 650b continuous with the orifice 650a which directs the molten plastic to runner pathways 648. The mold 610 is configured to receive molten plastic from such a nozzle. The mold 610 may also have a B-side 614 that is configured to translate with respect to the A-side 612.

The B-side 614 of the mold 610 may have a floating plate 624 slidably mounted with respect to a base plate 622. The base plate 622 may remain fixed in place, while the floating plate 624 may be configured to move a limited distance away from the base plate 622. The motion of the floating plate 624 with respect to the base plate 622 may be used to help remove a completed catheter assembly (not shown) from the mold 610. The base plate 622 may be configured to retain core pins 640 which may travel through the floating plate 624 and extend from the B-side 614 of the mold 610 into the A-side 612 of the mold 610 when the sides 612, 614 are mated for use. The floating plate 624 may additionally include runner paths 648 traveling away from a point 650b at which the runner paths 648 interface with the orifice 650a when the mold 610 is assembled. The floating plate 624 may further include core pins 640 extending from the base plate 622 through the floating plate 624. The floating plate 624 may additionally include a plate seal 632 to isolate the center of the mold 610 where the actual molding occurs.

The B-side 614 of the mold 610 may be configured to translate relative to the A-side 612 to allow selective mating or disengagement of the sides 612, 614. The A-side 612 may include a cavity plate 626. The A-side 612 of the mold 610 includes individual cavities 660 which receive the core pins 640 and leave space for receiving the molten plastic that defines the shape of the one-piece catheter assembly produced by the mold 610. In addition, the cavity plate 626 may include alignment bores 618 for receiving the pins 616 extending from the B-side 614 of the mold 610. One of ordinary skill in the art will add additional plates if needed to provide or support components of the mold such as the core pins, or to add components such as ejector pins.

The orientation of the A-side 612 and the B-side 614 of the mold 610 may be stabilized by a set of leader pins 616 extending from either the floating plate 624 or the base plate 622. The leader pins 616 are configured to pass through alignment bores 618 present in plates such as the floating plate 624 (if mounted to the base plate 622), or the cavity plate 626. These pins 616 stabilize the mold 610 to provide proper alignment of the individual plates 622, 624, and 626. In addition, the pins 616 allow translation of the individual plates 622, 624, and 626 relative to each other.

In use, the floating plate 624 is mated to the cavity plate 626 such that the cavities 660 define the space needed to produce the catheter assembly. The nozzle of an injection molding machine is attached to the cavity plate 626 having an orifice 650a. The injection molding machine injects molten plastic into the mold. For purposes of this discussion, the nozzle is connected to the orifice 650a of the cavity plate 626, and injects molten plastic into the orifice 650a. Having traveled through the orifice 650a, the molten plastic emerges on the surface of the floating plate 624. The molten plastic then travels across the floating plate 624 along runner pathways 648. The runner pathways 648 convey the molten plastic toward the core pins 640 and the cavities 660.

Referring now to Figure 9B, a partial plan view of the mold of Figure 9A is shown taken at the line 9B-9B of Figure 9A. Figure 9B shows the runner pathways 648 in detail. The runner pathways 648 may simply take the form of slots in the floating plate 624 of Figure 8A. As shown in Figure 9B, the runner pathways 648 may be substantially symmetrical, so that they can convey simultaneous flows of molten plastic toward the core pins 640 and the cavity 660. Similarly, the runner pathways 648 may open to the orifice 650A shown on Figure 9A at a region 650b configured to correspond to the orifice 650a with an orifice that may be of the same size. In addition, the runner pathways 648 may have the same length and cross sectional area. These characteristics help to provide uniform flows of molten plastic to the cavities 660.

One of skill in the art would understand that the runner pathways 648 shown in Figure 9B may be varied within the scope of the invention, and that in addition, alternate molding technologies may be used to produce the single-part and multi-part catheter assemblies of the invention without producing runners. In some specific examples, hot- and semi-hot-runner systems may be used with molds within the scope of the invention to produce the catheter assemblies of the invention. Such technologies often allow supply of molten plastic directly or near-directly to the mold cavity without utilizing a runner system. This avoids production of waste product and often shortens production cycle time.

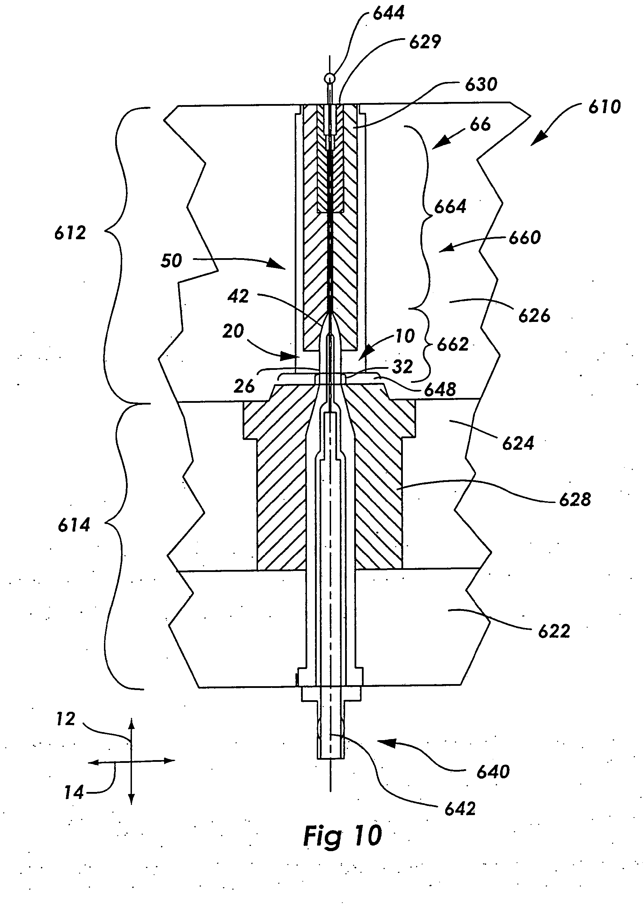

Referring now to Figure 10, a partial cross-sectional view of the mold 610 of Figure 9A is shown. In this Figure, the mold 610 has been assembled and injected with a suitable material as described above to form the single-material catheter assembly 10, and the catheter assembly 10 has been formed. As described above, the mold 610 includes an A-side 612 and a B-side 614 which are shown mated for use. In this Figure, the B-side 614 is shown to include a floating plate 624 and a base plate 622. In alternate embodiments of the mold 610, features and components of the A-side 612 and the B-side 614 may be exchanged. In addition, additional plates and components may be included as needed on either or both sides in order to support alternative catheter assembly 10 geometries, enhance use of the mold 610, and in some cases, to aid in ejection of the completed catheter assembly 10. The A-side 612 of the mold 610 is shown to include a cavity plate 626. In alternate embodiments of the mold 610, additional plates and components may be included as needed.

The cavity plate of the A-side 612 of the mold 610 includes a cavity 660 having a hub cavity region 662 and a catheter cavity region 664. During use, a core pin 640 may extend from the B-side 614 of the mold 610 into the hub cavity region 662, and then into the catheter cavity region 664. The core pin 640 defines the lumen of the resulting catheter assembly 10. In Figure 10, the core pin 640 is anchored to a proximal anchor 642 and a pilot 629. The proximal anchor 642 may be attached to the base plate 622 or to an assembly external to the mold 610 as shown in Figure 10. The pilot 629 attached to the distal end of the core pin 640 may be anchored to the cavity plate 626, or alternatively to an assembly outside of the mold 610 as shown in Figure 10.

The core pin 640 extends from the floating plate 624 into the cavity 660 of the cavity plate 626 in a manner such that molten plastic is unable to escape from the cavity 660. The core pin 640 may even extend from the floating plate 624 in an airtight manner, if desired. Alternatively, the core pin 640 and the floating plate may be made to fit together such that air is able to pass between them to exit the cavity 660.

In some embodiments, it will be desirable to apply a vacuum to the cavity 660 prior to injection of molten plastic into the cavity 660. This evacuates air from the cavity 660 to allow the molten plastic to entirely fill the cavity 660. The cavity plate 626 may thus include a vacuum channel 644 accessible from outside the mold 610. Vacuum fittings (not shown) may be attached to such a vacuum channel to draw air out of the cavity 660. If desired, the core pin 640 or pilot 629 may even be made slightly porous to expedite the expulsion of air from the cavity 660. The vacuum fitting may be coupled to a vacuum source, such as a vacuum pump, as is known in the art. In each of these situations, however, the core pin 640 is able to slide relatively freely through the floating plate 624 into the cavity 660 of the cavity plate 626.

In some embodiments of the mold 610 of the invention, it may be desirable to provide mold components which may be rapidly and inexpensively replaced in order to speed repairs and reduce repair costs. Thus, in some embodiments, the mold 610 may include modular blocks such as a taper lock stripper block 628, a modular pilot block 629, and a modular catheter block 630. These modular blocks 628, 629, 630 are mounted such that they are positioned in the appropriate plates 622, 624, 626 of the mold 610. One such mounting configuration of the modular blocks 628, 629, and 630 is shown in Figure 10. These modular blocks 628, 629, and 630 may permit rapid modification, repair, or replacement of various components of the floating plate 624, as well as the possibility of using the mold 610 to produce parts having different configurations. In one example, components of the mold 610 could be replaced to allow production of catheters of different sizes.

In one example, catheter block 630 may be substituted to allow the production of a catheter with a different length or width. The modular pilot block 629 may also be substituted to allow variation of the tip geometry of the catheter assembly produced by the mold. These modular blocks may be produced using methods known to one of ordinary skill in the art. The methods of the invention allow production of catheter assemblies having tip geometries equivalent to those previously produced using secondary tip processing methods.

The mold 610 may further include components to assist in the removal of the catheter assembly 10 from the mold 610, as well as components configured to remove any runner 648 formed during the process. Such removal methods include manual removal; mechanical removal by ejector pins, stripper plates, or robotic removal; or using other equipment and techniques known to one of ordinary skill in the art.

The hub and catheter cavity portions 662, 664 of the mold 610 form a cavity 660 into which a molten plastic may be injected. As illustrated in Figure 9B, in some embodiments of the mold 610 and methods of the invention, molten plastic is introduced into the cavity 660 via runners 648 which deliver molten plastic to at least two separate gate regions (not shown) of the cavity 660. This provides multiple flows of molten plastic into the cavity 660 in an even manner. These even flows may provide a high degree of molecular alignment in the longitudinal direction 12. In addition, the evenness of the flows may help to prevent deflection of the core pin 640.

In a next example, the invention further provides methods and apparatus for producing multi-part, and potentially multi-material integrated catheter assemblies. The following discussion proceeds with reference to the catheter assembly 110 of Figures 2A-3. One of ordinary skill in the art would be able to adapt this method to produce the catheter assemblies of the invention.

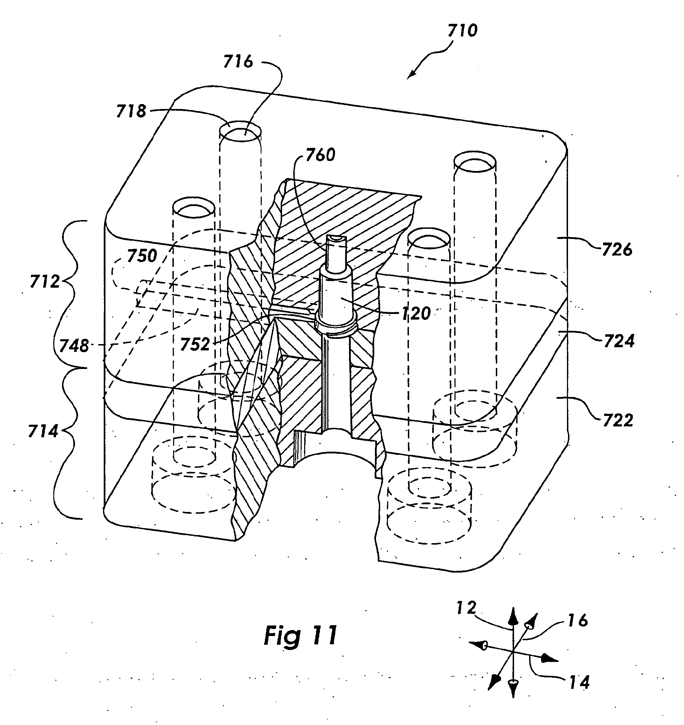

In many of these methods of the invention, either the catheter portion 150 or the hub portion 120 of the assembly 110 is first injection molded, and then in a subsequent step, the remaining component is overmolded onto the previously produced part. In the method of the invention illustrated in Figures 11-15, a sequential overmolding method is used in which the hub of a catheter assembly of the invention is molded in a first mold, removed from the first mold, inserted into a second mold, and overmolded with a catheter. One of ordinary skill in the art would understand that the method could be easily adapted to reverse the order in which the components are produced to produce the catheter portion first, and then to overmold the hub portion about it.

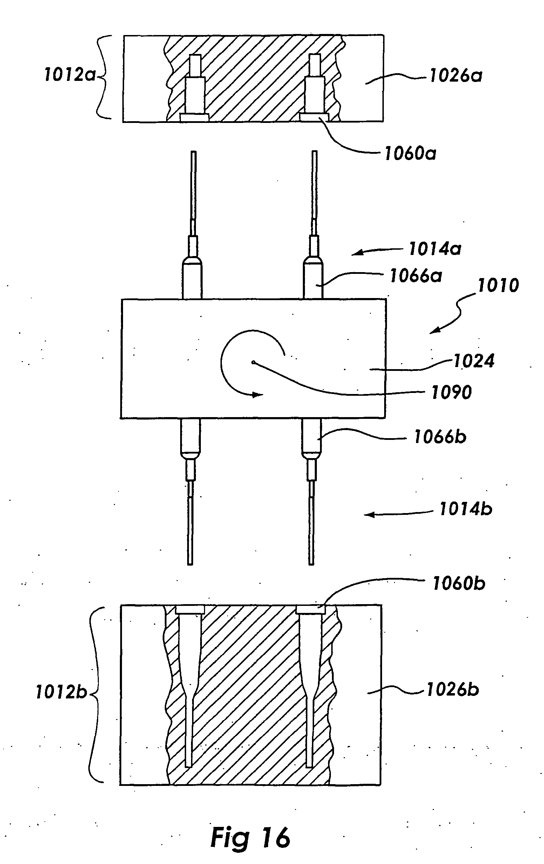

In addition to the above, in alternate embodiments of the invention, the overmolding process may be completed in a single mold which makes use of replaceable or rotating cores, rotating cavities on plates, or other similar technologies known to one of skill in the art in order to allow sequential production of the two components within a single mold. One mold suitable of functioning in such a fashion is shown in Figure 16. In still other embodiments of the invention, injection molding technologies may be utilized which allow injection of two or more molten plastics either simultaneously or in rapid succession. Other variations on these production methods that are understood by one of ordinary skill in the art are also included in the scope of the invention.

Referring first to Figure 11, a mold of the invention for use in sequential overmolding methods used to produce a multi-part integrated catheter assembly is shown. More specifically, a first mold 710 is shown for use in the production of an integrated two-piece catheter assembly of the invention such as catheter assembly 110 of Figures 2A-3. Figure 11 illustrates the configuration of a mold 710 configured to produce the hub portion 120 of the catheter assembly 110 of Figures 2A-3. This mold is used in the initial steps of a method for producing integrated two-piece catheters according to the invention to produce a hub portion 120 of the catheter assembly 110. According to the method, the hub portion 120 of the assembly 110 is first produced in a mold such as 710, and then the catheter portion 150 is overmolded onto the hub portion 120 to produce the final catheter assembly 110. One of ordinary skill in the art would understand that according to the methods of the invention, the mold 710 could instead be configured to produce the catheter portion 150 of the assembly 110, and that subsequently, the hub portion 120 could be molded thereto. In addition, one of skill in the art would understand that the mold and associated method could be modified to produce catheter assemblies including more than two components such as catheter assembly 910 of Figures 8A-8B.

The mold 710 includes an A-side 712 and a B-side 714 which mate as shown to provide a cavity 760 for injection molding a catheter hub portion. The B-side 714 includes a base plate 722, and a floating plate 724, while the A-side 712 includes a cavity plate 726. Other plates may be used in molds of the invention, as known in the art and described above, to add function to the mold. The plates 722, 724, 726 are aligned using leader pins 716 which extend from the base plate 722 through alignment bores 718 in the floating plate 724 and the cavity plate 726. In many embodiments of the mold 710 of the invention, the plates 724, 726 are slidable along the leader pins 716 such that the floating and cavity plates 724, 726 may be mated and separated during the steps of the method of the invention to provide a cavity 760 prior to injection of molten plastic, and then to allow removal of a molded component.

To produce a catheter hub 120 as shown in Figures 2A-3, the mold 710 of Figure 11 may further include a core pin (here omitted for clarity) which may extend from the B-side 714 of the mold 710 and project into the cavity 760 of the A-side 712. The core pin 740 projects into a cavity 760 of the cavity plate 726 and combines with the cavity 760 to define the shape of the component produced. The cavity 760 interfaces with a runner pathway 748 at a gate region 752. In this embodiment of the mold 710, a single gate region 752 is used. In alternate embodiments, multiple gate regions 752 may be used in order to vary the number of flows of plastic and to reduce the potential for deflection of the core pin 740 upon injection of molten plastic as discussed above. The runner pathway 748 exits the mold 710 at a sprue orifice 750. It is through this sprue orifice 750 that molten plastic is injected according to the method of the invention to produce the final molded part.

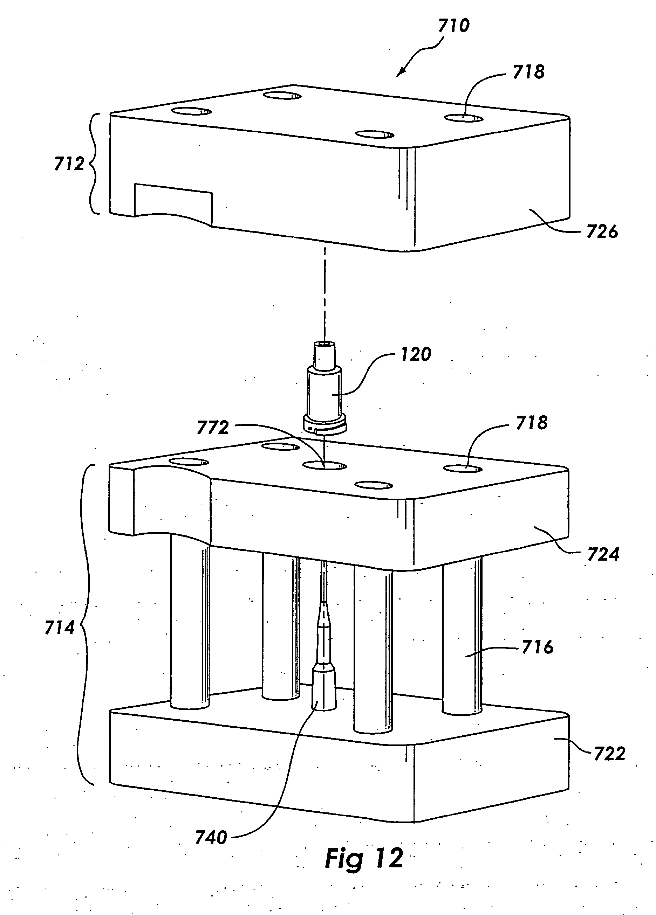

Referring now to Figure 12, an exploded perspective view of the mold 710 of Figure 11 is shown. Here, the B-side 714 is shown exploded, with the floating plate 724 shown to have translated in a longitudinal direction 12 along the leader pins 716. As above, the leader pins 716 are shown to pass through the floating plate 724 in alignment bores 718. Figure 12 further shows the A-side 712, including the cavity plate 726 detached from the mold 710. As shown, the floating plate 724 further contains a core pin orifice 772 through which the core pin 740 passes. The core pin 740 is shaped to define the lumen 146 of the catheter hub 120 of Figures 2A-3. Figure 12 further shows a catheter hub 120 according to the invention, shown ejected from the mold 710. The cavity plate 726 includes alignment bores 718 and is slidable along the leader pins 716 when attached to the B-side 714 of the mold 710.

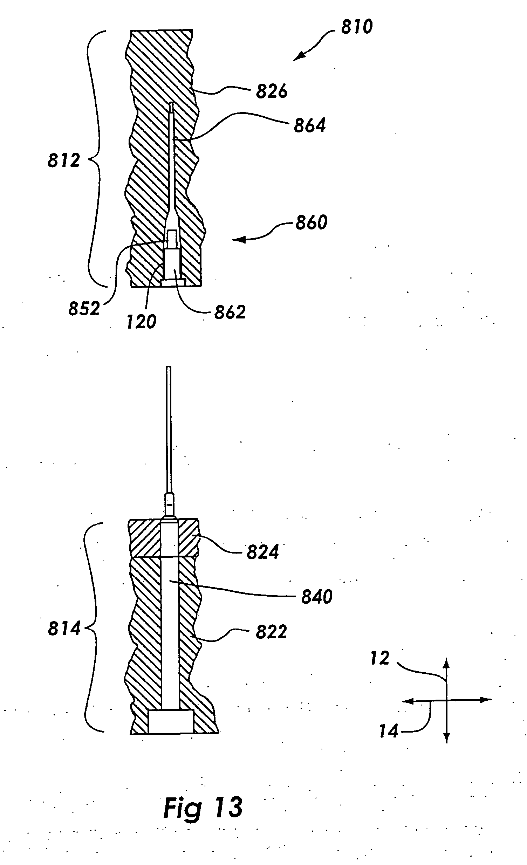

Referring now to Figure 13, a catheter overmold 810 is shown. In Figure 13, portions of the overmold 810 have been cut away for clarity. The catheter overmold 810 is used in the steps of the method of the invention to produce two-piece integrated catheter assemblies such as 110 shown in Figures 2A-3. The overmold 810 includes a base plate 822, a floating plate 824, and a cavity plate 826. The overmold 810 is shown open, with the A-side 812 separate from the B-side 814.

The overmold 810 includes a cavity 860 including a hub cavity portion 862 and a catheter cavity portion 864. The hub cavity portion 862 is generally positioned in the cavity plate 826. According to variations of the method of the invention, the two-piece integrated catheter assemblies of the invention may be produced by first molding the catheter hub 120 and then using an overmold such as 810 to integrally mold a catheter portion to the hub 120. Thus, in Figure 13, the overmold 810 is shown to include a catheter hub 120 molded using the initial steps of the method of the invention. Specifically, the catheter hub 120 has been molded, and then inserted into the hub cavity portion 862 of the cavity 860 of the overmold 810. As discussed above, one of ordinary skill in the art would understand that the order of molding could be varied. In addition, one of skill in the art would understand that the geometry of the catheter hub of the invention may be varied as taught above with reference to catheter assemblies 210, 310, 410, 510, and 910 of Figures 4-8.

With the catheter hub 120 placed in the hub cavity portion 862, the hub adapter portion 138 of the catheter hub 120 may be configured to extend into the catheter cavity portion 864 of the overmold 810 such that when the catheter portion 150 is molded about the hub portion 120, a secure joint 152 is formed. As discussed above, such a joint 152 may be formed simply by providing an adequate interface between the two portions 120, 150 of the catheter assembly 110 to allow the materials of the hub portion 120 and the catheter portion 150 to adhere. In addition, in some embodiments such as catheter assemblies 410 and 510 of Figures 6 and 7, it may be desirable to form a mechanical interlock. In either of these situations, the hub portion 120 extends into the catheter cavity portion 864. Those portions of the hub 120 which are present in the catheter cavity portion 864 of the cavity 860 may be directly overmolded with the molten plastic used to form the catheter portion of the catheter assembly.

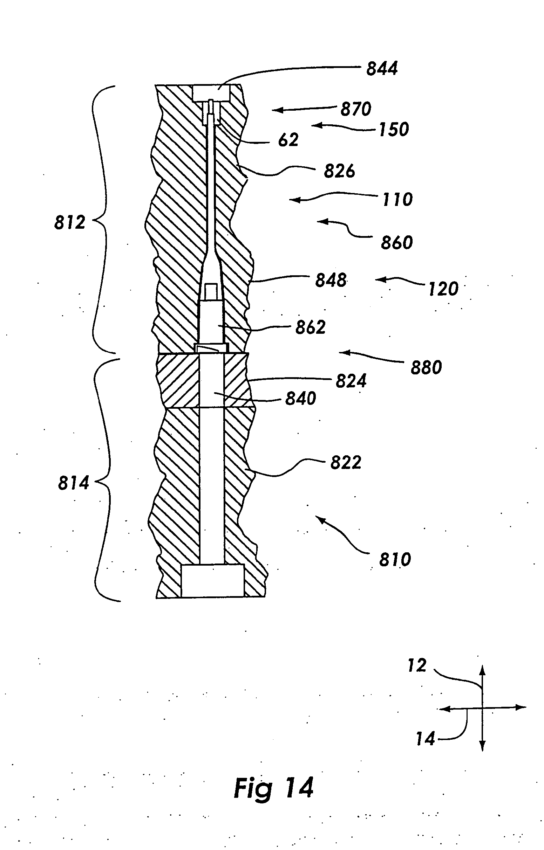

Referring now to Figure 14, the overmold 810 of Figure 13 is shown with the A-side 812 mated with the B-side 814 and with a catheter assembly 110 completely formed within. More specifically, the base plate 822 with the core pin 840 extending from it has been inserted into the cavity 860. As noted previously, the cavity 860 includes a hub cavity portion 862 and a catheter cavity portion 864. Prior to uniting the A-side 812 and the B-side 814, the pre-molded catheter hub 120 is inserted into the hub cavity portion 862 of the floating plate 824. The core pin 840 is configured to travel through the pre-molded catheter hub 120 to a specific point, at which the core pin 840 fits the hub 120 in a sealed manner such that when molten plastic is injected into the cavity 860, it is substantially directed into the catheter cavity portion 864.

The core pin 840 may be anchored to the base plate 822 in a variety of ways. In Figure 14, the attachment has been omitted for clarity. In some embodiments of the mold 810, the attachment may be similar to the proximal anchor 642 shown in Figure 10. In some instances, this attachment may be detachable to facilitate rapid repairs to or replacement of the core pin 840. The core pin 840 may then extend from the base plate 822 into the cavity 860 comprising the hub and catheter cavity portions 862, 864.

The cavity plate 826 may include a distal anchor 844, into which the core pin 840 extends when the A-side 812 and the B-side 814 of the mold 810 are mated, as shown in Figure 13. The distal anchor 844 may receive the distal end 870 of the core pin 840. The distal anchor 844 may support the distal end 870 against motion such as lateral deflection. The distal anchor 844 does not, however, pull the core pin 840 in the longitudinal direction 12. As a result, the core pin 840 is substantially untensioned. The distal end 870 and the distal anchor 844 may be precision formed such that the distal end 870 fits within the pilot distal anchor 844 with only a very small clearance, such as a clearance on the order of two ten-thousandths of an inch (0.0002"). Thus, the distal end 870 is precisely fixed in place, and molten plastic is unable to escape from the cavity 860 between the distal end 870 and the distal anchor 844.

The tip 62 of the catheter assembly 110 may be formed within the distal anchor 844. In the alternative to complete formation of the tip 62 within the distal anchor 844, the tip 62 may be created in roughened form in the injection molding process and further shaped through subsequent processing. For example, the tip 62 may be injection molded with a tubular shape similar to that of the remainder of the catheter portion 150. The tip 62 may then be tapered through reheating and shaping, mechanical cutting, or other similar operations.

The distal end 870 and the distal anchor 844 may be made to fit together such that air is able to pass between the distal anchor 844 and the distal end 870 to exit the cavity 860. In some embodiments, it will be desirable to apply a vacuum to the cavity 860 prior to injection of molten plastic into the cavity 860. This evacuates air from the cavity 860 to allow the molten plastic to entirely fill the cavity 860. The cavity plate 826 may thus include a vacuum channel (not shown) accessible from outside the mold 810. Vacuum fittings (not shown) may be attached to such a vacuum channel on the cavity plate 826 to be in gaseous communication with the distal anchor 844 to draw air out of the cavity 860 through the distal anchor 844. If desired, the distal anchor 844 may even be made slightly porous to expedite the expulsion of air from the cavity 860. The vacuum fitting may be coupled to a vacuum source, such as a vacuum pump, as is known in the art.

As described previously, the mold 810 may incorporate ejector pins capable of extending into the cavity 860 to aid in removal of a completed catheter assembly 110 from the mold 810. Additionally, ejector pins may be provided to eject the runners and the sprue from the mold 810. The runners are solidified plastic pieces formed in the runner pathways 848, and the sprue is a solidified plastic piece formed in a sprue orifice (not shown) of the cavity plate 826. The runners and the sprue are ejected to avoid interference with the next injection cycle; they may be discarded or recycled for use in future injection cycles.

In addition, as is taught in the art, the catheter assembly 110 may include a slight draft angle on the external and internal surfaces to provide a slightly tapered shape. The draft angle may, for example, be on the order of 0.125°. In the alternative, the catheter assembly 110 may be molded with a draft angle of 0°. In any case, the mold 810may be shaped to produce the desired, draft angle.

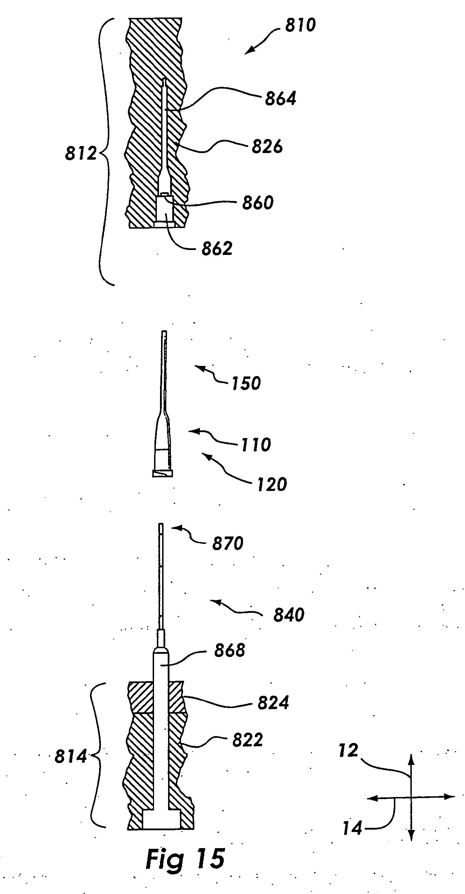

Referring now to Figure 15, the overmold 810 of Figures 13-14 is shown in an exploded view. Figure 15 thus shows the completed catheter assembly 110 ejected from the overmold 810. Thus, the base plate 822, with the core pin 840 extending from it is shown separated from the molded catheter assembly 110. The A-side 812 of the mold is shown separated, with the floating plate 824 separated from the cavity plate 826. As illustrated in Figures 9-12, the plates and sides of the overmold 810 may be separated while maintaining their alignment using a set of leader pins and alignment bores. Such alignment mechanisms are omitted from Figure 15 for clarity.

In some embodiments of the overmold 810 and method of the invention, it is desirable to provide multiple flows of molten plastic to the cavity 860 using multiple gate regions. In some specific embodiments, it is desirable to provide first and second flows of molten plastic to the cavity 860 to prevent deflection of the core pin 840. In some such embodiments, the first and second flows enter the cavity 860 from two sides of the catheter cavity region 864. In such embodiments, the first and second flows may converge in such a manner that the molten plastic is substantially evenly distributed about the circumference of the catheter cavity region 864. The molten plastic then flows through the catheter cavity region 864 substantially evenly. This helps the molten plastic to maintain a substantially even distribution about the core pin 840.

When such even flows are produced, the core pin 840 is under substantially the same pressure from all sides, and no significant deflection of the core pin 840 occurs. The molten plastic may continue to flow evenly to form the tip 62 of the catheter assembly 110. Injection molding machines used with the molds of the invention may be configured to rapidly step down the pressure of molten plastic within the molds of the invention such as 810 at a time selected to induce the molten plastic to stop flowing as soon as the catheter tip 62 of the catheter assembly 110 is formed.

These production methods may yield a catheter assembly 110 with a high degree of longitudinal molecular alignment, or molecular alignment in the longitudinal direction 12. Longitudinal molecular alignment may be desirable to prevent failure of the catheter assembly 110 under the stresses of insertion and subsequent use. The circumferential molecular alignment, or alignment in the lateral and transverse directions 14, 16, may be somewhat smaller than the longitudinal molecular alignment because the lateral and transverse directions 14, 16 are perpendicular to the direction in which molten plastic flows through the cavity 860 during the injection molding process.

The plastic that is used to form the catheter portion 150 may be optimized to the pressure and temperature characteristics of the molding process as well as to the geometry of the cavity 860. For example, the plastic may have a melt flow high enough to ensure that the entire cavity 860 is filled within a reasonable cycle time, yet low enough to avoid excessive flash or circulation within the cavity 860 after filling.

The method of the invention may be tuned such that the cavity 860 may be completely filled within a predetermined period of time. In some embodiments of the invention, such a time period may be from about 0.10 to about 0.20 seconds. After the cavity 860 has been filled, the molten plastic within the cavity 860 may be permitted to cool and solidify. Heat exchangers or the like, as known in the art, may be coupled to the mold 810 to facilitate cooling of the plastic within the cavity 860. Cooling may require a few seconds of time.

After the catheter portion 150 has been overmolded onto the hub portion 120 of the catheter assembly 110, the mold is partially disassembled to release the completed catheter assembly 110. In a first step of such disassembly, the core pin 840 is withdrawn from the A-side 812 of the overmold 810. Withdrawal of the core pin 840 generally results in removal of the completed catheter assembly 110 from the cavity 860, still attached to the core pin 840. The completed catheter assembly 110 may then be removed from the core pin using ejector pins, stripper blocks, or robotics which have been omitted from Figure 14 for clarity.

Referring now to Figure 16, yet another embodiment of a mold according to the invention is shown. More specifically, Figure 16 shows a top cutaway view of an alternate mold 1010 of the invention for use in multi-shot injection molding methods of producing single- or multi-component integrated catheter assemblies of the invention. The mold 1010 of Figure 16 is specifically configured to facilitate the production of multi-component integrated catheter assemblies. The mold 1010 includes multiple cavity plates 1026a, 1026b and a rotating base plate 1024. This effectively provides first and second A-sides 1012a, 1012b, and two B-sides 1014a, 1014b positioned on the rotating base plate 1024. The base plate 1024 may further include core pins 1066a for use in producing the hub portion of a catheter assembly, and core pins 1066b for use in producing the catheter portion of a catheter assembly. Generally, the core pins 1066a, 1066b are identical. The mold is thus configured to provide cavities 1060a and 1060b on two faces of the rotating base plate 1024. One of skill in the art could vary this design to utilize additional faces of the rotating base plate 1024.

The mold 1010 is shown in an exploded perspective view. The cavity plates 1026a, 1026b include cavities 1060a, 1060b. In this embodiment of the mold 1010, cavity plate 1026a is configured to produce a hub portion of a catheter assembly (not shown), and cavity plate 1026b is configured to accept the previously-molded hub portion and provide a cavity 1060b configured to overmold the catheter portion of a catheter assembly (not shown) onto the hub. One of skill in the art would understand that the mold could easily be configured to first produce the catheter portion and then allow overmolding of the hub portion of a catheter assembly within the scope of the invention.

In operation according to methods producing the hub portion first, the mold 1010 would receive a first injection of molten polymer or prepolymer into the first set of cavities 1060a. Upon filling and curing of the polymer, the plates of the mold 1026a, 1026b, 1024 would be separated. The base plate 1024 would then be rotated to position the completed hub portions (not shown) in alignment with cavities 1060b, after which the plates of the mold 1026a, 1026b, and 1024 would be mated again. When the plates are properly mated, molten polymer may be injected into both of the cavities 1060a, 1060b, thus simultaneously completing one set of catheter assemblies in cavities 1060b and forming the hub portions of another set in cavities 1060a.

The injection molding method and molds presented herein enable the production of catheter assemblies of the invention with a high degree of reliability, rapidity, and cost effectiveness. Through the use of a uniform distribution of molten plastic, the longitudinal molecular alignment of the plastic can be maintained, and excessive flash can be avoided.

The present invention may be embodied in other specific forms without departing from its structures, methods, or other essential characteristics as broadly described herein and claimed hereinafter. The described embodiments are to be considered in all respects only as illustrative, and not restrictive. The scope of the invention is, therefore, indicated by the appended claims, rather than by the foregoing description. All changes that come within the meaning and range of equivalency of the claims are to be embraced within their scope.

## Claims

1. A catheter assembly comprising:
a hub portion comprising an adapter and a first attachment face; and
a catheter portion comprising a tube and a second attachment face;
wherein the hub portion and the catheter portion are integrally formed by injection-molding, the hub portion and the catheter portion being attached to each other at their first and second attachment surfaces, respectively.

2. The catheter assembly of claim 1, wherein the hub portion and the catheter portion are formed of a single polymer.

3. The catheter assembly of claim 2, wherein the polymer is selected from the group consisting of polyurethane elastomer, polyester, polyethylene, polypropylene, polybutylene, polytetrafluoroethylene, fluorinated ethylene-propylene copolymer, silicone, polyether block amides, and poly vinyl chloride.

4. The catheter assembly of claim 3, wherein the polymer is a polyurethane elastomer.

5. The catheter assembly of claim 4, wherein the polymer is vialon™ or reduced molecular weight vialon™.

6. The catheter assembly of claim 1, wherein the hub portion and the catheter portion are formed of first and second polymers, respectively.

7. The catheter assembly of claim 6, wherein the first polymer is a substantially rigid polymer.

8. The catheter assembly of claim 7, wherein the first polymer is selected from the group consisting of nylon, polymethyl-methyacrylate, polyester, acrylo-nitrile butadiene styrene, polyurethane, polyethylene, polypropylene, polyether block amides, poly vinyl chloride, polycarbonate, acrylic, polystyrene, and polymethylpentene.

9. The catheter assembly of claim 8, wherein the first polymer is selected from the group consisting of polyethylene terephthalate, nylon 12 homopolymer, and nylon 12 copolymer.

10. The catheter assembly of claim 6, wherein the second polymer is a substantially flexible polymer.
